(19) Europäisches Patentamt
European Patent Office
Office européen des brevets

(11) **EP 4 647 005 A1**

(12) **EUROPEAN PATENT APPLICATION**
published in accordance with Art. 153(4) EPC

(43) Date of publication:
**12.11.2025 Bulletin 2025/46**

(21) Application number: **24764159.0**

(22) Date of filing: **27.02.2024**

(51) International Patent Classification (IPC):
*A61B 5/0537* (2021.01)   *A61B 5/00* (2006.01)
*A61B 5/389* (2021.01)   *A61B 5/053* (2021.01)
*G06F 3/01* (2006.01)

(52) Cooperative Patent Classification (CPC):
**A61B 5/00; A61B 5/053; A61B 5/0537;
A61B 5/389; A61B 5/4875; A61B 5/681**

(86) International application number:
**PCT/KR2024/002477**

(87) International publication number:
**WO 2024/181760 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **27.02.2023  KR 20230025867
07.04.2023  KR 20230046018**

(71) Applicant: **Samsung Electronics Co., Ltd.
Suwon-si, Gyeonggi-do 16677 (KR)**

(72) Inventors:
• **JEON, Taehan
Suwon-si Gyeonggi-do 16677 (KR)**

• **MIN, Jinhong
Suwon-si Gyeonggi-do 16677 (KR)**
• **OH, Youngjae
Suwon-si Gyeonggi-do 16677 (KR)**
• **CHOI, Hyoungseon
Suwon-si Gyeonggi-do 16677 (KR)**
• **KIM, Joonho
Suwon-si Gyeonggi-do 16677 (KR)**
• **KIM, Taeseon
Suwon-si Gyeonggi-do 16677 (KR)**
• **YOON, Seoyoung
Suwon-si Gyeonggi-do 16677 (KR)**

(74) Representative: **Gulde & Partner
Patent- und Rechtsanwaltskanzlei mbB
Berliner Freiheit 2
10785 Berlin (DE)**

(54) **ELECTRONIC DEVICE FOR MEASURING BIOIMPEDANCE AND METHOD FOR CONTROLLING SAME**

(57)    Disclosed is an electronic device. The electronic device includes a first electrode body configured to be in contact with a body of a user and comprising a first current electrode and a second current electrode symmetrically arranged in a first direction and a first voltage electrode and a second voltage electrode symmetrically arranged in the first direction, a second electrode body configured to be in contact with the body of a user and, and one or more processor configured to: obtain a first bioelectrical impedance through the first electrode body comprising a first current electrode and a second current electrode symmetrically arranged in a first direction and a first voltage electrode and a second voltage electrode symmetrically arranged in the first direction, obtain a second bioelectrical impedance through the second electrode body comprising a third current electrode and a fourth current electrode symmetrically arranged in a second direction and a third voltage electrode and a fourth voltage electrode symmetrically arranged in the second direction, identify one of the first bioelectrical impedance and the second bioelectrical impedance based on phases of each of the first bioelectrical impedance and the second bioelectrical impedance, obtain a total body water amount of the user using the identified bioelectrical impedance, and provide the obtained total body water amount.

**(Cont. next page)**

# FIG. 2

100

11                    10        12

| FIRST BIOELECTRICAL IMPEDANCE MEASUREMENT UNIT | ONE OR MORE PROCESSORS | SECOND BIOELECTRICAL IMPEDANCE MEASUREMENT UNIT |
|---|---|---|

| FIRST ELECTRODE BODY | | | | SECOND ELECTRODE BODY | | | |
|---|---|---|---|---|---|---|---|
| FIRST CURRENT ELECTRODE | FIRST VOLTAGE ELECTRODE | SECOND VOLTAGE ELECTRODE | SECOND CURRENT ELECTRODE | THIRD CURRENT ELECTRODE | THIRD VOLTAGE ELECTRODE | FOURTH VOLTAGE ELECTRODE | FOURTH CURRENT ELECTRODE |
| 111-1 | 112-1 | 112-2 | 111-2 | 121-1 | 122-1 | 122-2 | 121-2 |

110                                          120

## Description

### [Technical Field]

The disclosure relates to an electronic device and a control method thereof and, for example to, an electronic device for measuring bioelectrical impedance using a multi electrode and a control method thereof.

### [Background Art]

Bioelectrical impedance measurement technology is widely used as it is non-invasive, cost-saving, and has excellent stability in measuring total body water and body composition.

For example, in the related art, bioelectrical impedance has been measured using a device of which a location is fixed, but recently, bioelectrical impedance measurement technology is introduced to a portable device, a wearable device, or the like.

In the related art, the location-fixed device requires a limited posture to a user when measuring bioelectrical impedance, and requires various constraints such as prohibiting excessive exercise or maintaining empty stomach before measurement.

Bioelectrical impedance measurement technology introduced into a portable device or a wearable device is advantageous in that the total body water change or body composition change of a user may be monitored in real time, but it is disadvantageous in that a total body water change or the like is incorrectly measured.

For example, if a user is doing excessive exercise or after consuming a large amount of water when measuring bioelectrical impedance, there is a disadvantage in that the total body water fluctuation range measured by the device is large even though the total body water of the user cannot be rapidly changed for a short period of time.

Therefore, there has been a need for a method for measuring and providing total body water with high accuracy regardless of the user's activity state (for example, exercising state).

### [Disclosure]

### [Technical Solution]

An electronic device according to an example embodiment includes: a first electrode body configured to be in contact with a body and comprising: a first current electrode and a second current electrode symmetrically arranged in a first direction and a first voltage electrode and a second voltage electrode symmetrically arranged in the first direction, a second electrode body configured to be in contact with the body and comprising: a third current electrode and a fourth current electrode symmetrically arranged in a second direction and a third voltage electrode and a fourth voltage electrode symmetrically arranged in the second direction, and one or more processors configured to: obtain a first bioelectrical impedance through the first electrode body, obtain a second bioelectrical impedance through the second electrode body, identify one of the first bioelectrical impedance and the second bioelectrical impedance based on phases of each of the first bioelectrical impedance and the second bioelectrical impedance, obtain a total body water amount using the identified bioelectrical impedance, and provide the obtained total body water amount.

According to an example embodiment, a method of controlling an electronic device comprising a first electrode body and a second electrode body configured to be in contact with a body includes: obtaining a first bioelectrical impedance through the first electrode body comprising a first current electrode and a second current electrode symmetrically arranged in a first direction and a first voltage electrode and a second voltage electrode symmetrically arranged in the first direction, obtaining a second bioelectrical impedance through the second electrode body comprising a third current electrode and a fourth current electrode symmetrically arranged in a second direction and a third voltage electrode and a fourth voltage electrode symmetrically arranged in the second direction, identifying one of the first bioelectrical impedance and the second bioelectrical impedance based on phases of each of the first bioelectrical impedance and the second bioelectrical impedance, obtaining a total body water amount using the identified bioelectrical impedance, and providing the obtained total body water amount.

According to an example embodiment, a non-transitory computer-readable recording medium including a program which, when executed, causes an electronic device to perform a control method of a display device wherein the control method of an electronic device comprising a first electrode body and a second electrode body configured to be in contact with a body includes: obtaining a first bioelectrical impedance through the first electrode body comprising a first current electrode and a second current electrode symmetrically arranged in a first direction and a first voltage electrode and a second voltage electrode symmetrically arranged in the first direction, obtaining a second bioelectrical impedance through the second electrode body comprising a third current electrode and a fourth current electrode symmetrically arranged in a second direction and a third voltage electrode and a fourth voltage electrode symmetrically arranged in the second direction, identifying one of the first bioelectrical impedance and the second bioelectrical impedance based on phases of each of the first bioelectrical impedance and the second bioelectrical impedance, obtaining a total body water amount using the identified bioelectrical impedance, and providing the obtained total body water amount.

**[Brief Description of Drawings]**

The above and other aspects, features and advantages of certain embodiments of the present disclosure will be more apparent from the following detailed description, taken in conjunction with the accompanying drawings, in which:

FIG. 1 is a graph illustrating a change in bioelectrical impedance during resting and exercising of a user;
FIG. 2 is a block diagram illustrating an example configuration of an electronic device according to various embodiments;
FIG. 3 is a diagram illustrating a side view of an electronic device in contact with the body of a user according to various embodiments;
FIG. 4 is a diagram illustrating a plan view of an electronic device according to various embodiments;
FIG. 5 is a diagram illustrating an arrangement direction of an electrode based on the muscle cells included in a local area on the body of a user according to various embodiments;
FIG. 6 is a graph illustrating phases of each of a first bioelectrical impedance and a second bioelectrical impedance according to various embodiments;
FIG. 7 is a graph illustrating an example method for adjusting a total body water amount according to various embodiments;
FIG. 8 is a diagram illustrating an example electronic device including a plurality of electrode bodies according to various embodiments;
FIG. 9 is a graph illustrating an example method of obtaining and adjusting the total body water amount using a plurality of bioelectrical impedance according to various embodiments;
FIG. 10 is a diagram illustrating a side view illustrating a contact state between a user's body and the electronic device according to various embodiments;
FIG. 11 is a diagram illustrating an implementation example of the electronic device according to various embodiments; and
FIG. 12 is a flowchart illustrating an example method of operating the electronic device according to various embodiments.

**[Detailed Description of Exemplary Embodiments]**

Terms used in the present disclosure are briefly described, then disclosure will be described in greater detail with reference to the drawings.

The terms used in the present disclosure and the claims are general terms identified in consideration of the functions of the various embodiments of the disclosure. However, these terms may vary depending on intention, legal or technical interpretation, emergence of new technologies, and the like of those skilled in the related art. Also, some terms may be arbitrarily identified. Unless there is a specific definition of a term, the term may be construed based on the overall contents and technological common sense of those skilled in the related art.

Since the disclosure may be variously modified and have several embodiments, various non-limiting example embodiments of the disclosure will be illustrated in the drawings and be described in greater detail in the detailed description. However, it is to be understood that the disclosure is not limited to specific non-limiting example embodiments, but includes all modifications, equivalents, and substitutions without departing from the scope and spirit of the disclosure. When it is decided that a detailed description for the known art related to the disclosure may obscure the gist of the disclosure, the detailed description may be omitted.

As used herein, the terms "first," "second," or the like may identify corresponding components, regardless of importance of order, and are used to distinguish a component from another without limiting the components.

Singular forms are intended to include plural forms unless the context clearly indicates otherwise. It will be further understood that terms "include" or "formed of" used in the present disclosure specify the presence of features, numerals, steps, operations, components, parts, or combinations thereof mentioned in the present disclosure, but do not preclude the presence or addition of one or more other features, numerals, steps, operations, components, parts, or combinations thereof.

A term such as "module," "unit," "part," and so on is used to refer to an element that performs at least one function or operation, and such element may be implemented as hardware or software, or a combination of hardware and software. Further, other than when each of a plurality of "modules," "units," "parts," and the like must be realized in an individual hardware, the components may be integrated in at least one module or chip and be realized in one or more processors (not shown).

Hereinafter, various non-limiting example embodiments of the disclosure will be described in greater detail with reference to the accompanying drawings. However, the disclosure may be implemented in various different forms and is not limited to the example embodiments described herein. In addition, in the drawings, portions unrelated to the description may be omitted, and similar portions will be denoted by similar reference numerals throughout the disclosure.

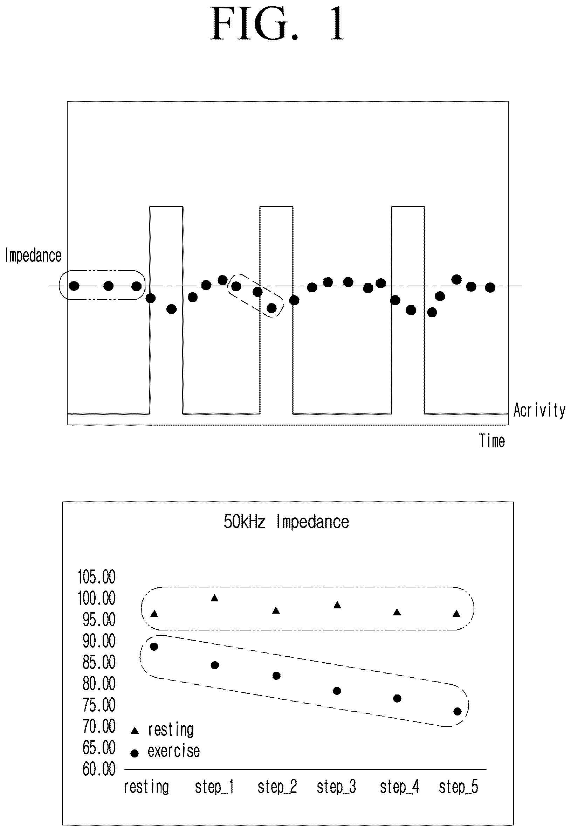

FIG. 1 is a graph illustrating a change in bioelectrical impedance during resting and exercising of a user.

The bioelectrical impedance technology (Bioelectrical Impedance Analysis, BIA) may refer, for example, to the technology of measuring a component (hereinafter, body component) of the human body using impedance measurable when microcurrent electrical signal is introduced to the human body.

The impedance may be determined as a vector sum of resistance and reactance. When a microcurrent electrical signal is applied to the human body, resistance is generated by components of total body water, adipose tissue, muscle cells, bone, or the like in the human body.

For example, the muscle cells include a lot of water so that a passage through which an electrical signal flows is wide (e.g., less resistance), and fat tissue includes less water, so that a passage through which an electrical signal flows is narrow (e.g., greater resistance).

Reactance may refer, for example, to an electrical resistance (e.g., reactance) occurring in a cell membrane when an electrical signal that flows along water in addition to cells in the human body meets a cell membrane and passes through the cell membrane to flow an electrical signal to water in the cell. Therefore, the reactance may represent the structural stability of the cell, unlike the resistance.

Impedance denotes human body resistance that occurs when microcurrent electrical signal flows in the human body, and is a vector sum of resistance and reactance.

Volume (V) may correspond to multiplication of length (L) and area (A) (V = L x A), and impedance (R) is inversely proportional to the cross-sectional area (A) and is proportional to the length (L) (R = L / A).

Impedance (R) may refer, for example, to bioelectrical impedance, length (L) may correspond to the height of the user, and volume (V) may correspond to the total body water amount of the human body, so the total body water amount is determined according to Equation 1 below.

[Equation 1]

$$\text{Total body water amount} = C \times L^2 / R$$

Here, C is a resistance per unit volume, and since a predetermined (e.g., specified) amount of electrolyte is dissolved per unit volume, the value is a constant value, and a user's height (L) may be measured, and thus a total body water amount may be obtained based on the bioelectrical impedance (R).

According to various examples, the body composition of the body includes total body water, protein, body fat, and mineral (bone), and the total body water and the protein are proportional to each other as main components of muscle cells (e.g., approximately, 72 : 37), and the weight of bone, which is the main component, is closely related to the muscle mass, so it is possible to know protein, minerals, and body fat mass (weight-fat free mass) (fat free mass = total body water + protein + minerals)), that is, all components of the body composition from the total body water amount. The weight is equal to the sum of the body components.

The electronic device according to various embodiments of the disclosure may be implemented with a device (e.g., wearable device) that is contactable with the human body of the user to measure the bioelectrical impedance.

For example, the electronic device may include an electrode body (for example, an electrode body used for electrocardiogram (ECG), or the like) coming in contact with local areas on various bodies such as a user's wrist, back of the hand, ankle, top of the foot, thigh, and the like, and may control the electrode body so as to flow a current (i) to a local area.

The current (i) passes a local area, the electronic device may measure the voltage V between the electrodes included in the electrode body and may obtain the bioelectrical impedance R according to the Ohm's law (R = V/i).

According to various examples, the electronic device may obtain a total body water amount based on the bioelectrical impedance, and may obtain protein, mineral, and a body fat mass as described above. The electronic device according to one or more examples may provide a ratio (total body water, protein, body fat, or mineral composition ratio) of a component of a body component of a user.

To identify the total body water amount with high accuracy and reliability, it is required to measure the bioelectrical impedance in the empty stomach state, resting state (e.g., before exercising).

As shown in the graph of FIG. 1, the bioelectrical impedance measured at a preset time interval is constantly maintained in a resting state, but the bioelectrical impedance is gradually lowered due to the activity of muscle cells in the body in an active state (for example, during exercising or immediately after exercising), and thus there may be a problem in that the electronic device inaccurately identifies the total body water amount (or the total body water, protein, body fat, and mineral composition ratio) of the body component of the user.

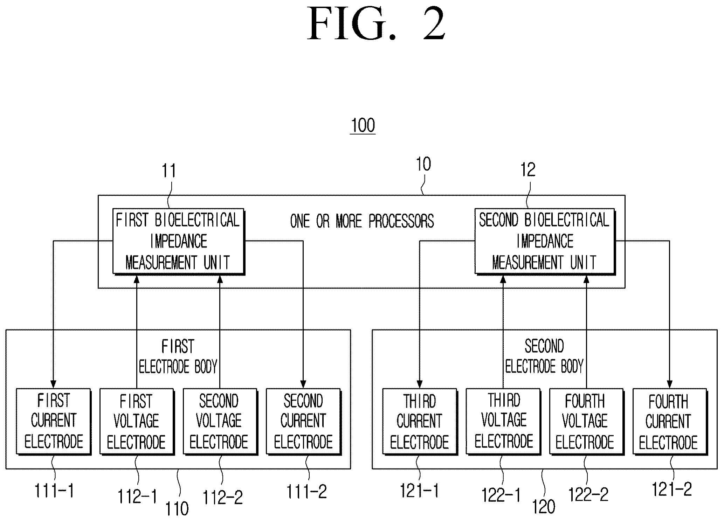

The electronic device according to various embodiments of the disclosure may appropriately adjust and provide the total body water amount even when bioelectrical impedance is incorrectly measured according to the activity of muscle cells. A description thereof will be provided in greater detail below with reference to FIG. 2.

FIG. 2 is a block diagram illustrating an example configuration of an electronic device according to various embodiments.

Referring to FIG. 2, the electronic device 100 includes a first electrode body 110, a second electrode body 120, and one or more processors (e.g., including processing circuitry) 10.

The electronic device 100 according to various embodiments may include, for example, and without limitation, a wearable device and a portable device. The wearable device may refer, for example, to a device made of a flexible material (for example, silicone rubber, fiber, etc.) and worn by a user or in contact with a part of a user's body. For example, various types of devices may be included in a wearable device, such as, for example, and without limitation, a watch, garment, shoes, glove, glasses, hat, accessory (e.g., ring), etc., that may be worn on the body of a human or animal. However, this is merely an example, and the embodiments are not limited thereto.

According to various embodiments, the electronic device 100 may include at least one of, for example, and without limitation, a television (TV), a user terminal device, a tablet personal computer (PC), a mobile phone, a video phone, an e-book reader, a desktop PC, a laptop PC, a netbook computer, a workstation, a server, a personal digital assistant (PDA), a portable multimedia player (PMP), an MP3 player, a medical device, a camera, a virtual reality (VR) device, or a wearable device. Herein, a wearable device may include at least one of an accessory type (e.g., a watch, a ring, a bracelet, an ankle bracelet, a necklace, a pair of glasses, a contact lens or a head-mounted-device (HMD)); a fabric or a garment-embedded type (e.g.: electronic cloth); skin-attached type (e.g., a skin pad or a tattoo); or a bio-implantable circuit. In various embodiments, the electronic device 100 may include at least one of, for example, a TV, a digital video disk (DVD) player, an audio system, a refrigerator, air-conditioner, a cleaner, an oven, a microwave, a washing machine, an air purifier, a source device (e.g., a set-top box, a cloud server, an over-the-top (OTT) media service server, etc.), a home automation control panel, a security control panel, a media box (e.g., APPLE TV™, or GOOGLE TV™), LED S-Box, a game console (e.g., XBOX™, PLAYSTATION™, NINTENDO SWITCH™), an electronic dictionary, an electronic key, a camcorder, or an electronic frame.

In various embodiments, the electronic device 100 may include at least one of a variety of medical devices (e.g., various portable medical measurement devices such as a blood glucose meter, a heart rate meter, a blood pressure meter, or a temperature measuring device), a total body water measuring device, magnetic resonance angiography (MRA), magnetic resonance imaging (MRI), computed tomography (CT), a capturing device, or an ultrasonic wave device, etc.), a navigation system, a global navigation satellite system (GNSS), an event data recorder (EDR), a flight data recorder (FDR), an automotive infotainment devices, a marine electronic equipment (e.g., marine navigation devices, gyro compasses, etc.), avionics, a security device, a car head unit, industrial or domestic robots, a drone, an automated teller machine (ATM) of financial institutions, a point of sale (POS) of a store, or an Internet of Things (IoT) device (e.g., light bulbs, sensors, sprinkler devices, fire alarms, thermostats, street lights, toasters, exercise equipment, hot water tanks, heater, boiler, etc.). Hereinbelow, for convenience, and without limitation, the electronic device 100 is assumed to be a wearable device (e.g., smart watch).

The electronic device 100 according to various embodiments of the disclosure may include the first electrode body 110 and the second electrode body 120 in contact with the body of a user.

The first electrode body 110 according to various embodiments may include the first current electrode 111-1 and the second current electrode 111-2 symmetrically disposed in the first direction and the first voltage electrode 112-1 and the second voltage electrode 112-2 symmetrically disposed in the first direction.

The second electrode body 120 according to various embodiments may include a third current electrode 121-1 and a fourth current electrode 121-2 arranged symmetrically to each other in a second direction; and a third voltage electrode 122-1 and a fourth voltage electrode 122-2 symmetrically arranged in a second direction.

The one or more processors 10 may include various processing circuitry (as used herein, including the claims, the term "processor" may include various processing circuitry, including at least one processor, wherein one or more processors processor may be configured to perform the various functions described herein) and control overall operations of the electronic device 100. According to an embodiment, the one or more processors 10 may, for example, and without limitation, be implemented with a digital signal processor (DSP), a microprocessor, and a time controller (TCON) which process a digital image signal, but this is not limited thereto. The processor 10 may include, for example, and without limitation, one or more among a central processing unit (CPU), a micro controller unit (MCU), a micro processing unit (MPU), a controller, an application processor (AP), a communication processor (CP), an advanced reduced instruction set computing (RISC) machine (ARM) processor, an artificial intelligence (AI) processor, or the like, or may be defined as a corresponding term. The one or more processors 10 may be implemented in a system on chip (SoC) type or a large scale integration (LSI) type which a processing algorithm is built therein or in a field programmable gate array (FPGA) type. The one or more processors 10 may perform various functions by executing computer executable instructions stored in a memory.

The one or more processors 10 may be implemented as one or more processors. The one or more processors may include one or more of a Central Processing Unit (CPU), a Graphics Processing Unit (GPU), an Accelerated Processing Unit (APU), a Many Integrated Core (MIC), a Digital Signal Processor (DSP), a Neural Processing Unit (NPU), a hardware accelerator, or a machine learning accelerator. The one or more processors 10 may control one or any combination of other components of the electronic device and may perform operations or data processing relating to the communication. The one or more processors 10 may execute one or more programs or instructions stored in the memory. For example, the one or more processors 10 may perform a method in accordance with various embodiments of the disclosure by executing one or more instructions stored in the memory.

When a method according to various embodiments of the disclosure includes a plurality of operations, a plurality of operations may be performed by one processor or may be performed by a plurality of processors. For example, when a first operation, a second operation, and a third operation are performed by a method according to various embodiments, all of the first operation, the second operation, and the third operation may be performed by the first processor, the first operation and the second operation may be performed by a first processor (e.g., a general purpose processor), and the third operation may be performed by a second processor (e.g., an artificial intelligence dedicated processor).

The one or more processors 10 may be implemented as a single core processor including one core, or may be implemented as one or more multicore processors including a plurality of cores (for example, homogeneous multi-cores or heterogeneous multi-cores). When one or more processors is implemented as a multi-core processor, each of the plurality of cores included in the multi-core processor may include a processor internal memory such as a cache memory and an on-chip memory, and a common cache shared by the plurality of cores may be included in the multi-core processor. In addition, each of a plurality of cores (or a part of a plurality of cores) included in the multi-core processor may independently read and perform a program command for implementing a method according to various embodiments of the disclosure, and may read and perform a program command for implementing a method according to various embodiments of the disclosure in connection with all (or a part of) a plurality of cores.

When the method according to various embodiments of the disclosure includes a plurality of operations, the plurality of operations may be performed by one core among a plurality of cores included in the multi-core processor or may be performed by the plurality of cores. For example, when a first operation, a second operation, and a third operation are performed by a method according to various embodiments, all the first operation, second operation, and third operation may be performed by a first core included in the multi-core processor, and the first operation and the second operation may be performed by a first core included in the multi-core processor and the third operation may be performed by a second core included in the multi-core processor.

In various embodiments of the disclosure, the one or more processors may include, for example, a system-on-chip (SoC), a single core processor, a multi-core processor, or a core included in a single core processor or a multi-core processor in which one or more processors and other electronic components are integrated, wherein the core may be implemented as a CPU, a GPU, an APU, a MIC, a DSP, an NPU, a hardware accelerator, or a machine learning accelerator, but embodiments of the disclosure are not limited thereto.

The one or more processors 10 according to various embodiments may include a first bioelectrical impedance measurement unit (e.g., including various processing circuitry and/or executable program instructions) 11 and a second bioelectrical impedance measurement unit (e.g., including various processing circuitry and/or executable program instructions) 12.

The bioelectrical impedance measurement unit 11 may control the first electrode body 110 so that current i flows between the first current electrode 111-1 and the second current electrode 111-2.

According to various embodiments, current flows from a first current electrode 111-1 to the second current electrode 111-2, and the first bioelectrical impedance measurement unit 11 may measure a voltage (v) in a loop formed from the first voltage electrode 112-1 to the second voltage electrode 112-2. A first bioelectrical impedance measurement unit 11 according to various embodiments may measure a human body resistance in a first direction, that is, a first bioelectrical impedance.

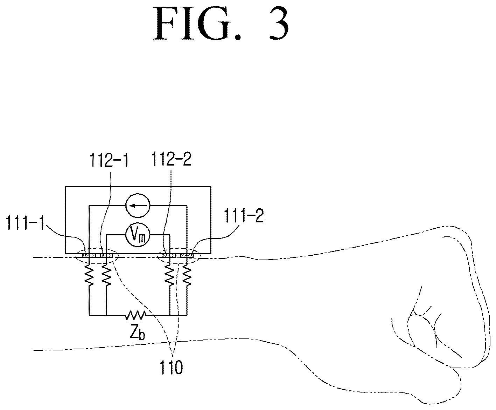

A description will be provided in greater detail below with reference to FIG. 3.

FIG. 3 is a diagram illustrating a side view of an electronic device in contact with the body of a user according to various embodiments.

Referring to FIG. 3, according to various embodiments, the electronic device 100 includes one surface (hereinafter, rear surface) in contact with the body of a user and the other surface (hereinafter, the front surface) may include a display (not shown) for providing various information (for example, blood glucose information, body composition information, etc.), and the first conductor 110 and the second conductor 120 may be provided on the rear surface.

According to various embodiments, the first conductor 110 may include the first current electrode 111-1 and the second current electrode 111-2 arranged symmetrically to the first current electrode 111-1 with respect to the center of the rear surface.

According to various embodiments, the first bioelectrical impedance measurement unit 11 may control the first electrode body 110 such that a microcurrent (i) flows between the first current electrode 111-1 and the second current electrode 111-2.

According to various embodiments, the first bioelectrical impedance measurement unit 11 may measure a voltage (v) in a loop formed from the first voltage electrode 112-1 to the second voltage electrode 112-2, and may measure a human body resistance (Zb) in a first direction, that is, the first bioelectrical impedance according to a microcurrent (i) and a voltage (v).

The first voltage electrode 112-1 is disposed to be adjacent to the first current electrode 111-1 with an interval, and the second voltage electrode 112-2 may be arranged symmetrically to the first voltage electrode 112-1 with respect to the center of the rear surface. In addition, according to various embodiments, the second voltage electrode 112-2 may be disposed to be adjacent to the second current electrode 111-2 with an interval.

According to various embodiments, the first current electrode 111-1 and the second current electrode 111-2 are symmetrically arranged in a first direction, and the first voltage electrode 112-1 and the second voltage electrode 112-2 are symmetrically arranged in a first direction, and thus the first bioelectrical impedance measurement unit 11 may obtain a bioelectrical impedance (hereinafter, referred to as the first bioelectrical impedance) corresponding to a first direction of muscle cells (that is, muscle tissue) included in a local area in which the electronic device 100 is in contact on the body of the user.

Returning to FIG. 2, the second bioelectrical impedance measurement unit 12 may control the second electrode body 120 so that current i flows between the third current electrode 121-1 and the fourth current electrode 121-2.

According to various embodiments, the current i may flow from the third current electrode 121-1 to the fourth current electrode 121-2, and the second bioelectrical impedance measurement unit 12 may measure the voltage v in a loop formed from the third voltage electrode 122-1 to the fourth voltage electrode 122-2. A second bioelectrical impedance measurement unit 11 according to various embodiments may measure a human body resistance in a second direction, that is, a second bioelectrical impedance.

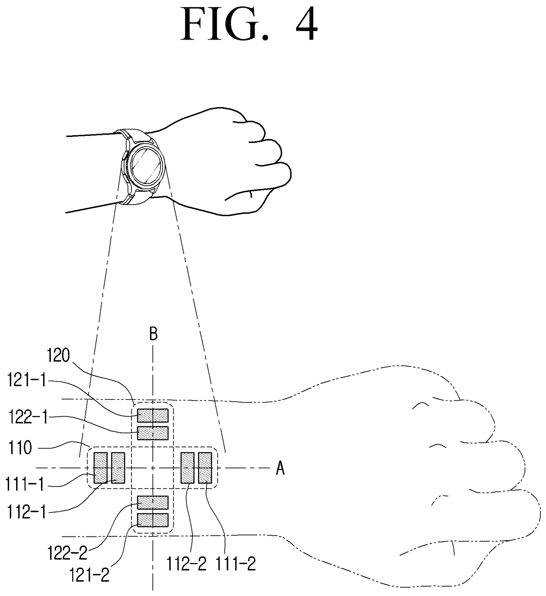

A description will be provided in greater detail below with reference to FIG. 4.

FIG. 4 is a diagram illustrating a plan view of an electronic device according to various embodiments.

Referring to FIG. 4, according to various embodiments, the second conductor 120 may include a third current electrode 121-1 and a fourth current electrode 121-2 arranged symmetrically to the third current electrode 121-1 with respect to the center of the rear surface.

According to various embodiments, the second bioelectrical impedance measurement unit 12 may control the second electrode body 120 so that fine current I flows between the third current electrode 121-4 and the fourth current electrode 121-2.

According to various embodiments, the second bioelectrical impedance measurement unit 12 may measure the voltage (v) in a loop formed from the third voltage electrode 122-1 to the fourth voltage electrode 122-2, and may measure the human body resistance in the second direction B according to the microcurrent (i) and the voltage (v), that is, the second bioelectrical impedance.

The third voltage electrode 122-1 may be disposed to be adjacent to the third current electrode 121-1 with an interval, and the fourth voltage electrode 122-2 may be arranged symmetrically to the third voltage electrode 132-1 with reference to the center of the rear surface. In addition, according to various embodiments, the fourth voltage electrode 122-2 may be may be disposed to be adjacent to the fourth current electrode 121-2 with an interval.

According to various embodiments, FIG. 4 shows that each of the third voltage electrodes 122-1 and 122-2 is disposed closer to the center of the rear surface than the third current electrode 121-1 and the fourth current electrode 121-2, but this is merely an example and is not limited thereto.

For example, the third current electrode 121-1 and the fourth current electrode 121-2 may be arranged closer to the center of the rear surface than the third voltage electrode 122-1 and the fourth voltage electrode 122-2, respectively. Alternatively, each of the first current electrode 111-1 and the second current electrode 111-2 may be disposed closer to the center of the rear surface than the first voltage electrode 112-1 and the second voltage electrode 112-2.

Referring to FIG. 4, according to various embodiments, the third current electrode 121-1 and the fourth current electrode 121-2 are arranged symmetrically to each other in the second direction B, and the third voltage electrode 122-1 and the fourth voltage electrode 122-2 are symmetrically arranged in the second direction B, so that the second bioelectrical impedance measurement unit 12 may obtain the bioelectrical impedance (hereinafter, the second bioelectrical impedance) corresponding to the second direction B of the muscle cell (e.g., muscle tissue) included in the local area in which the electronic device 100 is in contact with the body of the user. According to various embodiments, a first direction (A) and a second direction (B) may be orthogonal.

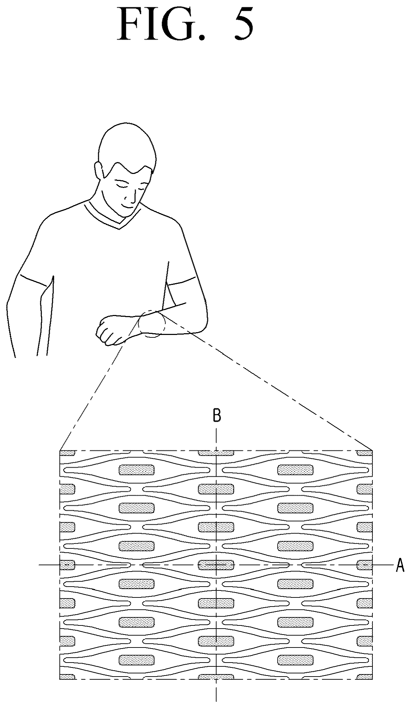

A description will be provided in greater detail below with reference to FIG. 5.

FIG. 5 is a diagram illustrating an arrangement direction of an electrode based on the muscle cells included in a local area on the body of a user according to various embodiments.

Referring to FIG. 5, the first direction A according to various embodiments may be horizontal to the muscle cells and the second direction B may be vertical to the muscle cells.

Bioelectrical impedance may be a vector sum of resistance and reactance. The reactance may refer to a resistance generated due to a cell membrane when a micro current flowing along extracellular water in the body passes through the cell membrane to flow into the intracellular water, unlike the resistance generated when the micro current (i) flows along the intracellular water and the extracellular water.

According to various embodiments, since the first direction (A) is horizontal to muscle cells, the bioelectrical impedance (e.g., the first bioelectrical impedance) corresponding to the first direction is less affected by the resistance (e.g., reactance) due to the cell membrane of the muscle cells.

According to various embodiments, the second direction (B) is vertical to the muscle cells, and the bioelectrical impedance (e.g., the second bioelectrical impedance) corresponding to the second direction may be largely affected by cell membrane of the muscle cells.

To identify a total body water amount according to bioelectrical impedance with high accuracy and reliability, a measurement of bioelectrical impedance at an empty stomach state, a resting state (e.g., before exercising) is required. In an activity state, a bioelectrical impedance corresponding to a specific direction (for example, a direction perpendicular to muscle cells) cells in the body is measured to be slightly lower due to the activity of muscle, and thus there is a problem in that a total body water amount is incorrectly identified. For example, when the total body water amount is identified using only the bioelectrical impedance corresponding to the second direction B, the total body water amount may be very incorrectly identified in an active state (for example, during exercise or immediately after exercise).

The electronic device 100 according to various embodiments of the disclosure may identify a plurality of bioelectrical impedance using a multi-electrode (for example, the first electrode body 110 and the second electrode body 120), and identify a total body water amount using at least one of the plurality of bioelectrical impedance.

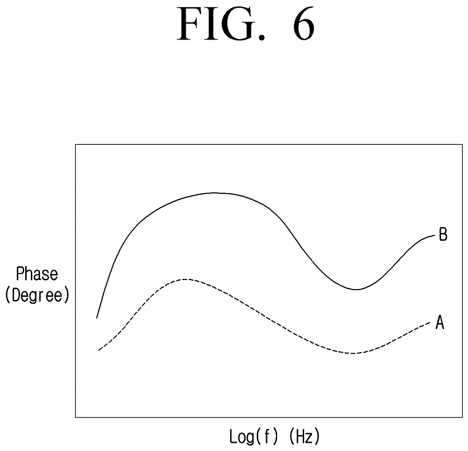

A description will be provided in greater detail below with reference to FIG. 6.

FIG. 6 is a graph illustrating phases of each of a first bioelectrical impedance and a second bioelectrical impedance according to various embodiments.

Referring to FIG. 6, one or more processors 10 may control the first electrode body 110 and the second electrode body 120 so that microcurrent (i) corresponding to each of a plurality of frequencies flows.

According to various embodiments, the degree of cell penetration is different for each frequency of a microcurrent (i), and a microcurrent of 5 kHz among frequencies of 5 to 200 kHz does not pass through a cell membrane so as to measure extracellular water (ECW), and a high-frequency microcurrent of 100 kHz or higher passes through the cell membrane so as to measure total body water (TBW).

The one or more processors 10 according to an embodiment of the disclosure may obtain a bioelectrical impedance (that is, the first bioelectrical impedance) corresponding to a first direction A and the bioelectrical impedance (that is, the second bioelectrical impedance) corresponding to a second direction B, and identify a total body water amount of the user using one of the first bioelectrical impedance and the second bioelectrical impedance based on a phase of each of the first bioelectrical impedance and the second bioelectrical impedance.

Referring to FIG. 6, one of the first bioelectrical impedance and the second bioelectrical impedance may have a large influence of resistance (e.g., reactance) due to the cell membrane of muscle cells than the other. For example, a bioelectrical impedance corresponding to a second direction B perpendicular to muscle cells may be measured relatively more than a bioelectrical impedance corresponding to a first direction A horizontal to muscle cells.

According to various embodiments, when the user is in an active state, not in a resting state, the phase of the second bioelectrical impedance is measured relatively larger than the phase of the first bioelectrical impedance due to the activity of muscle cells, and when the one or more processors 10 measure the total body water amount based on the second bioelectrical impedance, there is a problem in that the accuracy of the total body water amount decreases.

According to various embodiments, the one or more processors 10 may identify a bioelectrical impedance having a relatively low phase among a plurality of bioelectrical impedance, and measure a total body water amount based on the identified bioelectrical impedance. For example, when the user is in an active state, not in a resting state, the phase of the second bioelectrical impedance is measured relatively larger than the phase of the first bioelectrical impedance due to the activity of muscle cells, and the one or more processors 10 may measure the total body water amount based on the first bioelectrical impedance with a small influence of resistance (e.g., reactance) due to the cell membrane of muscle cells.

According to various embodiments, the one or more processors 10 may identify muscle activation information based on a second bioelectrical impedance since the phase of the second bioelectrical impedance is measured relatively larger than the phase of the first bioelectrical impedance due to the activity of muscle cells when the user is in an active state.

The degree of activity of muscles may correspond to the degree of activity of muscle cells included in the local area to which the electronic device 100 contacts on the body of the user.

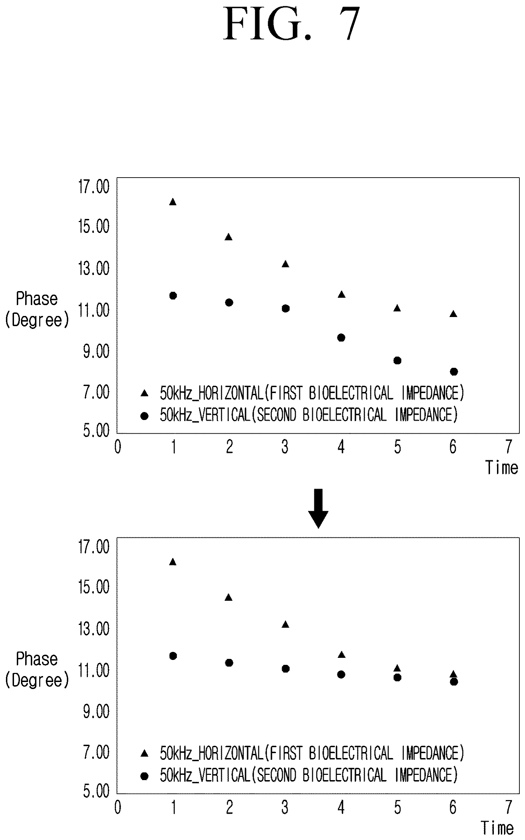

FIG. 7 is a graph illustrating an example method for adjusting a total body water amount according to various embodiments.

Referring to FIG. 7, the electronic device 100 may obtain a plurality of bioelectrical impedance at preset time intervals, and may monitor a fluctuation in the total body water amount based on the plurality of bioelectrical impedance, or may provide a fluctuation in the total body water amount to the user.

When a user is an active state according to various embodiments, each of a plurality of bioelectrical impedances may fluctuate (or change) according to the degree of activity of muscle cells.

Referring to FIG. 7, one or more processors 10 may control the first electrode body 110 to obtain a first bioelectrical impedance at a preset time interval, and identify a phase change corresponding to the first bioelectrical impedance.

The one or more processors 10 may control the second electrode body 120 to obtain a second bioelectrical impedance at a predetermined time interval, and identify a phase change corresponding to the second bioelectrical impedance.

Since the total body water amount of the human body does not rapidly change within a short time, each of the first bioelectrical impedance and the second bioelectrical impedance obtained at preset time intervals should be limited in fluctuation range, but when the degree of activity of muscle cells increases due to the user's activity state (for example, intense exercise, etc.), the first bioelectrical impedance and the second bioelectrical impedance may be significantly changed.

The one or more processors 10 may have a problem of measuring and providing an incorrect total body water amount based on first and second bioelectrical impedance that have changed significantly, despite the fact that the actual total body water amount in the user's body has not been significantly changed.

For example, the bioelectrical impedance (e.g., the second bioelectrical impedance) corresponding to a direction perpendicular to muscle cells (e.g., the second direction) changes sensitively depending on the degree of activity of muscle cells since a microcurrent (i) is measured by passing through a cell membrane of a plurality of muscle cells, and when one or more processors 10 identify a change in the total body water amount based on the second bioelectrical impedance, even though the total body water amount has not changed significantly, there is a problem in that the total body water amount that varies greatly within a short time (hereinafter, referred to as a threshold time) is provided.

The one or more processors 10 according to various embodiments of the disclosure may obtain and provide a change in total body water amount based on the first bioelectrical impedance obtained at a preset time interval when a phase change corresponding to a first bioelectrical impedance is lower than a phase change corresponding to the second bioelectrical impedance.

The one or more processors 10 according to various embodiments may adjust a change in the total body water amount based on the phase change corresponding to the second bioelectrical impedance obtained at a preset time interval.

Referring to FIG. 6, when a phase change corresponding to the second bioelectrical impedance exceeds a threshold range within a threshold time (that is, when the fluctuation range of a plurality of second bioelectrical impedance obtained at a preset time interval exceeds a threshold range), the one or more processors 10 may adjust the total body water amount obtained based on the first bioelectrical impedance and provide the adjusted total body water amount.

The one or more processors 10 according to various embodiments do not have significant fluctuations in the total body water amount of a human body when the fluctuation range of a plurality of second bioelectrical impedance obtained at preset time intervals exceeds a threshold range, but since each of the first bioelectrical impedance and the second bioelectrical impedance rapidly changes due to the activity of muscle cells, the total body water amount provided to the user may be maintained.

For example, when the fluctuation range of the plurality of second bioelectrical impedance obtained at preset time intervals exceeds a threshold range, the one or more processors 10 may ignore the gradual decrease of the plurality of first bioelectrical impedances obtained at preset time intervals and maintain the total body water amount provided to the user.

For example, the one or more processors 10 may obtain a total body water amount based on the first bioelectrical impedance having a small fluctuation range according to the degree of activity of muscle cells in a user's resting state. The one or more processors 10 may adjust a fluctuation range of the first bioelectrical impedance (for example, ignore a change in a first bioelectrical impedance or apply a weight value between 0 and 1 to a fluctuation width of the first bioelectrical impedance) based on the second bioelectrical impedance having a large fluctuation range according to the degree of activity of muscle cells in an active state of a user, and obtain a total body water amount based on the adjusted first bioelectrical impedance.

Accordingly, the one or more processors 10 may obtain a plurality of bioelectrical impedance using each of a plurality of electrodes so that the total body water amount is not incorrectly measured due to the degree of activity of muscle cells even though the actual total body water amount of the user has not changed, and may obtain a total body water amount based on the adjusted bioelectrical impedance after adjusting the remaining bioelectrical impedance (for example, the first bioelectrical impedance) based on any one bioelectrical impedance among the plurality of bioelectrical impedances.

Any one bioelectrical impedance may include a bioelectrical impedance having a large fluctuation range according to the degree of activity of muscle cells, and the remaining other bioelectrical impedance may be a bioelectrical impedance having a small fluctuation range according to the degree of activity of muscle cells.

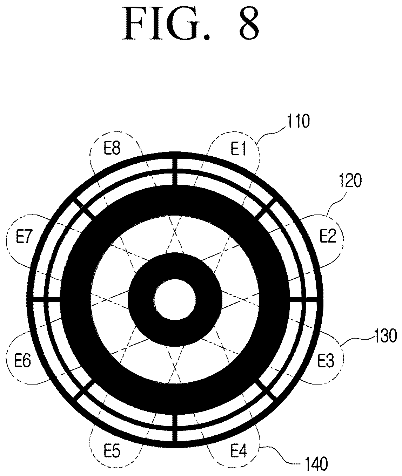

FIG. 8 is a diagram illustrating an example electronic device including a plurality of electrode bodies according to various embodiments.

In various embodiments described above, for convenience of description, the electronic device 100 includes the first electrode body 110 and the second electrode body 120, but this is an example, and the electronic device 100 may include a plurality of electrode bodies.

FIG. 8 is a diagram illustrating a plurality of electrode bodies provided in a rear surface of the electronic device 100.

In various examples, the first electrode body 110 may include the first current electrode 111-1 and the first voltage electrode 112-1 located on E1, and the second current electrode 111-2 and a second voltage electrode 112-2 located on E5. Here, E1 and E5 may be symmetrical to each other with respect to the center of the rear surface. The first electrode body 110 may measure a human body resistance in a first direction (hereinafter, referred to as the first bioelectrical impedance).

The second electrode body 120 may include the third current electrode 121-1 located on the E2, and the fourth current electrode 121-2 and the fourth voltage electrode 122-2 located on the E6. Here, E2 and E6 may be symmetrical to each other with respect to the center of the rear surface. The second electrode body 120 may measure a human body resistance in a second direction (hereinafter, referred to as a second bioelectrical impedance).

The third electrode body 130 may include a fifth current electrode and a fifth voltage electrode located in E3, and a sixth current electrode and a sixth voltage electrode located in E7. Here, the E3 and the E7 may be symmetrical to each other with respect to the center of the rear surface. The third electrode body 130 may measure a third bioelectrical impedance (hereinafter, referred to as a third bioelectrical impedance) in a third direction.

The fourth electrode body 140 may include a seventh current electrode and a seventh voltage electrode located on the E4, and an eighth current electrode and an eighth voltage electrode located in the E8. Here, the E4 and the E8 may be symmetrical to each other with respect to the center of the rear surface. The fourth electrode body 140 may measure a fourth bioelectrical impedance (hereinafter, referred to as a fourth bioelectrical impedance) in a fourth direction.

The one or more processors 10 according to various embodiments of the disclosure may obtain a plurality of bioelectrical impedance according to various directions, identify a bioelectrical impedance having a relatively large phase among the plurality of bioelectrical impedance as a bioelectrical impedance having a large fluctuation range according to the degree of activity of muscle cells, and identify a bioelectrical impedance having a relatively small phase as a bioelectrical impedance having a small fluctuation width according to the degree of activity of muscle cells.

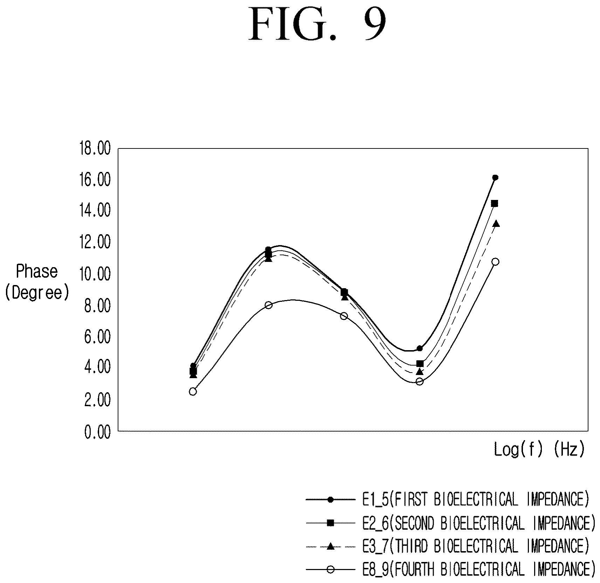

The description will be provided in greater detail below with reference to FIG. 9.

FIG. 9 is a graph illustrating an example method of obtaining and adjusting the total body water amount using a plurality of bioelectrical impedance according to various embodiments.

Referring to FIG. 9, in a plurality of frequencies, phases of each of a plurality of bioelectrical impedances (e.g., first to fourth bioelectrical impedance) may be different.

The one or more processors 10 may identify a bioelectrical impedance (first bioelectrical impedance E1_E5 of FIG. 9) having a relatively large phase among a plurality of bioelectrical impedance as a bioelectrical impedance having a large fluctuation range according to the degree of activity of muscle cells.

The one or more processors 10 may identify bioelectrical impedance (fourth bioelectrical impedance (E4_E8) in FIG. 9) having a relatively small phase among a plurality of bioelectrical impedances as bioelectrical impedance having a small fluctuation range according to the degree of activity of muscle cells.

The one or more processors 10 may obtain a total body water amount based on a fourth bioelectrical impedance. The one or more processors 10 according to various embodiments may adjust a phase change of a fourth bioelectrical impedance for a threshold time when a fluctuation range of a first bioelectrical impedance exceeds a threshold range within a threshold time, and obtain a total body water amount based on the adjusted fourth bioelectrical impedance.

According to various embodiments of the disclosure, the one or more processors 10 may obtain a human body resistance according to each of various directions (for example, first to fourth directions) using a plurality of electrode bodies 110 to 140.

The one or more processors 10 may identify human resistance that sensitively reacts to muscle cells according to the contact state of the electronic device 100, and human resistance that does not react sensitively. The one or more processors 10 may obtain a total body water amount based on a human body resistance that is not sensitive to muscle cells, but may adjust the total body water amount based on the fluctuation range of the human body resistance sensitively reacting to muscle cells, and then provide the total body water amount to a user.

In FIG. 9, the first to fourth electrode bodies 110 to 140 are examples for convenience of description, and the number of electrode bodies that may be provided in the electronic device 100 is not limited.

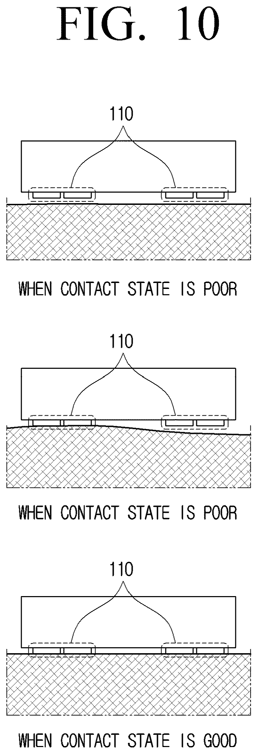

FIG. 10 is a diagram illustrating various side views illustrating a contact state between a user's body and the electronic device according to various embodiments.

Referring to FIG. 10, the one or more processors 10 may obtain a human body resistance according to various directions (for example, first to fourth directions) using a plurality of electrode bodies 110 to 140.

The one or more processors 10 may obtain the bioelectrical impedance from each of a plurality of electrode bodies 110 to 140 and may obtain the total body water amount using at least one of a plurality of bioelectrical impedances.

The one or more processors 10 according to various embodiments may obtain a total body water amount by measuring bioelectrical impedance from the remaining electrode bodies even though a bioelectrical impedance is not measured from any one electrode body among the plurality of electrode bodies 110 to 140 according to a contact state failure between the electronic device 100 and the user's body.

According to one or more examples, one or more processors 10 may obtain a total body water amount based on a second bioelectrical impedance when a second bioelectrical impedance is received from the second electrode body 120 even though the first bioelectrical impedance is not received from the first electrode body 110.

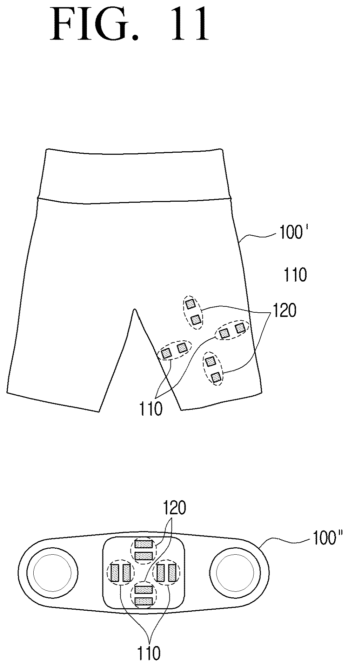

FIG. 11 is a diagram illustrating an implementation example of the electronic device according to various embodiments.

Referring to FIG. 11, the electronic device 100 may be implemented as a fabric or clothing-integrated device 100' (e.g., electronic garment) or body-attached device 100" (e.g.: skin pad, tattoos), or the like.

According to various examples, when the electronic device 100 is implemented as a clothing-integrated device, and when a user exercises while wearing the clothing-integrated device, the one or more processors 10 may appropriately adjust an error between the measured total body water amount and an actual total body water amount due to the activity of muscle cells even when the degree of activity of muscle cells increases according to the activity state of the user.

In addition, one or more processors 10 may identify the degree of activity of the muscle cells included in the local area with which the electronic device is in contact using the bioelectrical impedance sensitive to the degree of activity of the muscle cells.

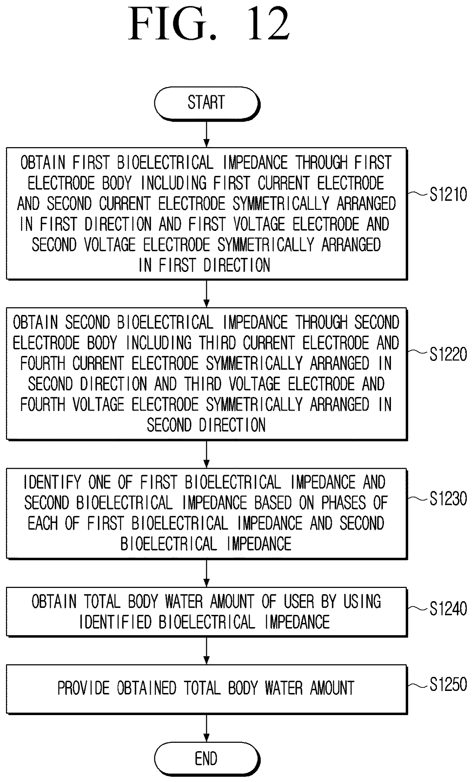

FIG. 12 is a flowchart illustrating an example method of controlling the electronic device according to various embodiments. A method of controlling the electronic device including the first electrode body and the second electrode body in contact with a body of a user according to various embodiments includes, obtaining a first bioelectrical impedance through the first electrode body comprising a first current electrode and a second current electrode symmetrically arranged in a first direction and a first voltage electrode and a second voltage electrode symmetrically arranged in the first direction in operation S1210.

The method may include obtaining a second bioelectrical impedance through the second electrode body comprising a third current electrode and a fourth current electrode symmetrically arranged in a second direction and a third voltage electrode and a fourth voltage electrode symmetrically arranged in the second direction in operation S1220.

The method may include identifying one of the first bioelectrical impedance and the second bioelectrical impedance based on phases of each of the first bioelectrical impedance and the second bioelectrical impedance in operation S1230.

The method may include obtaining a total body water amount of the user using the identified bioelectrical impedance in operation S1240.

The method may include providing the obtained total body water amount in operation S1250.

The identifying in operation S1230 according to various embodiments may include identifying the first bioelectrical impedance, based on the phase corresponding to the first bioelectrical impedance being lower than the phase corresponding to the second bioelectrical impedance.

The method according to various embodiments may further include identifying a muscle activity degree of the user based on the second bioelectrical impedance, and the muscle activity degree may correspond to the activity degree of muscle cells included in a local area on the body with which the electronic device is in contact.

The obtaining the first bioelectrical impedance in operation S1210 according to various embodiments may include identifying a phase change corresponding to the first bioelectrical impedance by obtaining the first bioelectrical impedance at a preset time interval through the first electrode body, and the obtaining the second bioelectrical impedance in operation S1220 may include identifying a phase change corresponding to the second bioelectrical impedance by obtaining the second bioelectrical impedance at a preset (e.g., specified) time interval through the second electrode body, the identifying one of the first bioelectrical impedance and the second bioelectrical impedance in operation S 1230 may include, based on a phase change corresponding to the first bioelectrical impedance being lower than a phase change corresponding to the second bioelectrical impedance, identifying the first bioelectrical impedance, the obtaining the total body water amount in operation S 1240 may include obtaining a fluctuation of the total body water amount based on the first bioelectrical impedance obtained at the preset time interval, and the providing in operation S1250 may include providing a fluctuation of the total body water amount.

The obtaining the fluctuation of the total body water amount according to various embodiments may include, based on the phase change corresponding to the second bioelectrical impedance exceeding a threshold range, adjusting the total body water amount obtained based on the first bioelectrical impedance.

The obtaining the fluctuation of the total body water amount according to various embodiments may include, based on the phase change corresponding to the second bioelectrical impedance exceeding the threshold range within the threshold time, ignoring a fluctuation of the total body water amount according to the phase change corresponding to the first bioelectrical impedance during the threshold time.

The first bioelectrical impedance according to various embodiments may be obtained through the first voltage electrode and the second voltage electrode symmetrically arranged to correspond to a horizontal direction of a muscle cell included in a local area of the body with which the electronic device is in contact, the second bioelectrical impedance may be obtained through the third voltage electrode and the fourth voltage electrode symmetrically arranged to correspond to a vertical direction of the muscle cell, the first direction may correspond to the horizontal direction of the muscle cell, and the second direction may correspond to the vertical direction of the muscle cell.

The electronic device according to various embodiments may further include a third electrode body remotely disposed in each of the first electrode body and the second electrode body, and the method may further include obtaining a third bioelectrical impedance through the third electrode body, and the obtaining the total body water amount comprises, based on a phase corresponding to the third bioelectrical impedance being relatively lower than phases each corresponding to the first bioelectrical impedance and the second bioelectrical impedance, obtaining the total body water amount based on the third bioelectrical impedance.

The electronic device according to various embodiments may be implemented as a wearable device comprising a display on a front surface, and each of the first electrode body and the second electrode body may be remotely disposed at a rear surface of the wearable device, and the providing comprises providing the total water amount through the display.

The various embodiments may be applicable to not only electronic devices but also all types of electronic devices including an electrode body.

The various example embodiments described above may be implemented in a recordable medium which is readable by computer or a device similar to computer using software, hardware, or the combination of software and hardware. In some cases, embodiments described herein may be implemented by the processor itself. According to a software implementation, embodiments such as the procedures and functions described herein may be implemented with separate software modules. Each of the above-described software modules may perform one or more of the functions and operations described herein.

The computer instructions for performing the processing operations of the electronic device 100 according to the various embodiments described above may be stored in a non-transitory computer-readable medium. The computer instructions stored in this non-transitory computer-readable medium cause the above-described specific device to perform the processing operations of the electronic device 100 according to the above-described various embodiments when executed by the processor of the specific device.

The non-transitory computer readable medium may refer, for example, to a medium that stores data, such as a register, a cache, a memory or etc., and is readable by a device. For example, the aforementioned various applications, instructions, or programs may be stored in the non-transitory computer readable medium, for example, a compact disc (CD), a digital versatile disc (DVD), a hard disc, a Blu-ray disc, a universal serial bus (USB), a memory card, a read only memory (ROM), and the like, and may be provided.

While the disclosure has been illustrated and described with reference to various embodiments thereof, it will be understood by those of skilled in the art that various changes in form and details may be made therein without departing from the spirit and scope of the disclosure including the appended claims and their equivalents. It will also be understood that any of the embodiment(s) described herein may be used in conjunction with any other embodiment(s) described herein.

**Claims**

1. An electronic device comprising:

   a first electrode body configured to be in contact with a body and comprising a first current electrode and a second current electrode symmetrically arranged in a first direction and a first voltage electrode and a second voltage electrode symmetrically arranged in the first direction;
   a second electrode body configured to be in contact with the body and comprising a third current electrode and a fourth current electrode symmetrically arranged in a second direction and a third voltage electrode and a fourth voltage electrode symmetrically arranged in the second direction; and
   one or more processor configured to:

      obtain a first bioelectrical impedance through the first electrode body,
      obtain a second bioelectrical impedance through the second electrode body,
      identify one of the first bioelectrical impedance and the second bioelectrical impedance based on phases of each of the first bioelectrical impedance and the second bioelectrical impedance,
      obtain a total body water amount using the identified bioelectrical impedance, and
      provide the obtained total body water amount.

2. The electronic device of claim 1, wherein the one or more processors are configured to identify the first bioelectrical impedance, based on the phase corresponding to the first bioelectrical impedance being lower than the phase corresponding to the second bioelectrical impedance.

3. The electronic device of claim 2, wherein the one or more processors are configured to identify a muscle activity degree based on the second bioelectrical impedance,
   wherein the muscle activity degree corresponds to the activity degree of muscle cells included in a local area on the body with which the electronic device is in contact.

4. The electronic device of claim 1, wherein the one or more processors are configured to:

identify a phase change corresponding to the first bioelectrical impedance by obtaining the first bioelectrical impedance at a preset time interval through the first electrode body, identify a phase change corresponding to the second bioelectrical impedance by obtaining the second bioelectrical impedance at a preset time interval through the second electrode body, based on a phase change corresponding to the first bioelectrical impedance being lower than a phase change corresponding to the second bioelectrical impedance, obtain a fluctuation of the total body water amount based on the first bioelectrical impedance obtained at the preset time interval, and provide a fluctuation of the total body water amount.

5. The electronic device of claim 4, wherein the one or more processors are configured to, based on the phase change corresponding to the second bioelectrical impedance exceeding a threshold range, adjust and provide the total body water amount obtained based on the first bioelectrical impedance.

6. The electronic device of claim 5, wherein the one or more processors are configured to, based on the phase change corresponding to the second bioelectrical impedance exceeding the threshold range within the threshold time, ignore a fluctuation of the total body water amount according to the phase change corresponding to the first bioelectrical impedance during the threshold time.

7. The electronic device of claim 1, wherein the first bioelectrical impedance is obtained through the first voltage electrode and the second voltage electrode symmetrically arranged to correspond to a horizontal direction of a muscle cell included in a local area of the body with which the electronic device is in contact,

wherein the second bioelectrical impedance is obtained through the third voltage electrode and the fourth voltage electrode symmetrically arranged to correspond to a vertical direction of the muscle cell,
wherein the first direction corresponds to the horizontal direction of the muscle cell, and
wherein the second direction corresponds to the vertical direction of the muscle cell.

8. The electronic device of claim 1, further comprising:

a third electrode body remotely disposed in each of the first electrode body and the second electrode body,
wherein the one or more processors are configured to:

obtain a third bioelectrical impedance through the third electrode body, and
based on a phase corresponding to the third bioelectrical impedance being relatively lower than phases each corresponding to the first bioelectrical impedance and the second bioelectrical impedance, obtain the total body water amount based on the third bioelectrical impedance.

9. The electronic device of claim 1, wherein the electronic device comprises a wearable device comprising a display on a front surface,

wherein each of the first electrode body and the second electrode body is disposed at a rear surface of the wearable device, and
wherein the one or more processors provide the total water amount through the display.

10. A method of controlling an electronic device comprising a first electrode body and a second electrode body in contact with a body, the method comprising:

obtaining a first bioelectrical impedance through the first electrode body comprising a first current electrode and a second current electrode symmetrically arranged in a first direction and a first voltage electrode and a second voltage electrode symmetrically arranged in the first direction;
obtaining a second bioelectrical impedance through the second electrode body comprising a third current electrode and a fourth current electrode symmetrically arranged in a second direction and a third voltage electrode and a fourth voltage electrode symmetrically arranged in the second direction;
identifying one of the first bioelectrical impedance and the second bioelectrical impedance based on phases of each of the first bioelectrical impedance and the second bioelectrical impedance;
obtaining a total body water amount using the identified bioelectrical impedance; and
providing the obtained total body water amount.

11. The method of claim 10, wherein the identifying comprises identifying the first bioelectrical impedance, based on the phase corresponding to the first bioelectrical impedance being lower than the phase corresponding to the second bioelectrical impedance.

12. The method of claim 11, wherein the method further comprises identifying a muscle activity degree based on the second bioelectrical impedance,
wherein the muscle activity degree corresponds to the activity degree of muscle cells included in a local area on the body with which the electronic device is in contact.

13. The method of claim 10, wherein the obtaining the first bioelectrical impedance comprises identifying a phase change corresponding to the first bioelectrical impedance by obtaining the first bioelectrical impedance at a preset time interval through the first electrode body,

wherein the obtaining the second bioelectrical impedance comprises identifying a phase change corresponding to the second bioelectrical impedance by obtaining the second bioelectrical impedance at a preset time interval through the second electrode body,
wherein identifying one of the first bioelectrical impedance and the second bioelectrical impedance comprises based on a phase change corresponding to the first bioelectrical impedance being lower than a phase change corresponding to the second bioelectrical impedance, identifying the first bioelectrical impedance,
wherein the obtaining the total body water amount comprises obtaining a fluctuation of the total body water amount based on the first bioelectrical impedance obtained at the preset time interval, and
wherein the providing comprises providing a fluctuation of the total body water amount.

14. The method of claim 13, wherein the obtaining the fluctuation of the total body water amount comprises, based on the phase change corresponding to the second bioelectrical impedance exceeding a threshold range, adjusting the total body water amount obtained based on the first bioelectrical impedance.

15. The method of claim 14, wherein the obtaining the fluctuation of the total body water amount comprises, based on the phase change corresponding to the second bioelectrical impedance exceeding the threshold range within the threshold time, ignoring a fluctuation of the total body water amount according to the phase change corresponding to the first bioelectrical impedance during the threshold time.

# FIG. 1

# FIG. 2

100

10

11 ONE OR MORE PROCESSORS 12

| | | |
|---|---|---|
| FIRST BIOELECTRICAL IMPEDANCE MEASUREMENT UNIT | | SECOND BIOELECTRICAL IMPEDANCE MEASUREMENT UNIT |

FIRST ELECTRODE BODY

SECOND ELECTRODE BODY

| FIRST CURRENT ELECTRODE | FIRST VOLTAGE ELECTRODE | SECOND VOLTAGE ELECTRODE | SECOND CURRENT ELECTRODE | THIRD CURRENT ELECTRODE | THIRD VOLTAGE ELECTRODE | FOURTH VOLTAGE ELECTRODE | FOURTH CURRENT ELECTRODE |
|---|---|---|---|---|---|---|---|
| 111-1 | 112-1 | 112-2 | 111-2 | 121-1 | 122-1 | 122-2 | 121-2 |

110

120

EP 4 647 005 A1

# FIG. 3

# FIG. 4

# FIG. 5

# FIG. 6

Phase
(Degree)

B

A

Log(f) (Hz)

# FIG. 7

# FIG. 8

# FIG. 9

# FIG. 10

110

WHEN CONTACT STATE IS POOR

110

WHEN CONTACT STATE IS POOR

110

WHEN CONTACT STATE IS GOOD

# FIG. 11

# FIG. 12

START

OBTAIN FIRST BIOELECTRICAL IMPEDANCE THROUGH FIRST ELECTRODE BODY INCLUDING FIRST CURRENT ELECTRODE AND SECOND CURRENT ELECTRODE SYMMETRICALLY ARRANGED IN FIRST DIRECTION AND FIRST VOLTAGE ELECTRODE AND SECOND VOLTAGE ELECTRODE SYMMETRICALLY ARRANGED IN FIRST DIRECTION ~S1210

OBTAIN SECOND BIOELECTRICAL IMPEDANCE THROUGH SECOND ELECTRODE BODY INCLUDING THIRD CURRENT ELECTRODE AND FOURTH CURRENT ELECTRODE SYMMETRICALLY ARRANGED IN SECOND DIRECTION AND THIRD VOLTAGE ELECTRODE AND FOURTH VOLTAGE ELECTRODE SYMMETRICALLY ARRANGED IN SECOND DIRECTION ~S1220

IDENTIFY ONE OF FIRST BIOELECTRICAL IMPEDANCE AND SECOND BIOELECTRICAL IMPEDANCE BASED ON PHASES OF EACH OF FIRST BIOELECTRICAL IMPEDANCE AND SECOND BIOELECTRICAL IMPEDANCE ~S1230

OBTAIN TOTAL BODY WATER AMOUNT OF USER BY USING IDENTIFIED BIOELECTRICAL IMPEDANCE ~S1240

PROVIDE OBTAINED TOTAL BODY WATER AMOUNT ~S1250

END

## INTERNATIONAL SEARCH REPORT

| | International application No. |
|---|---|
| | **PCT/KR2024/002477** |

| A. | CLASSIFICATION OF SUBJECT MATTER |
|---|---|

**A61B 5/0537**(2021.01)i; **A61B 5/00**(2006.01)i; **A61B 5/389**(2021.01)i

According to International Patent Classification (IPC) or to both national classification and IPC

| B. | FIELDS SEARCHED |
|---|---|

Minimum documentation searched (classification system followed by classification symbols)

A61B 5/0537(2021.01); A61B 5/00(2006.01); A61B 5/02(2006.01); A61B 5/053(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체 임피던스(bioimpedance), 전극(electrode), 전압(voltage), 전류(current), 위상 (phase), 체수분(body water)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2021-0361185 A1 (BETH ISRAEL DEACONESS MEDICAL CENTER, INC.) 25 November 2021 (2021-11-25)<br>See paragraph [0096], claim 15 and figure 4. | 1-15 |
| A | KR 10-2022-0009884 A (SAMSUNG ELECTRONICS CO., LTD.) 25 January 2022 (2022-01-25)<br>See claim 1. | 1-15 |
| A | KR 10-2022-0082909 A (TRUSTEES OF DARTMOUTH COLLEGE) 17 June 2022 (2022-06-17)<br>See claims 1 and 2 and figures 3a and 3b. | 1-15 |
| A | KR 10-2020-0012598 A (SAMSUNG ELECTRONICS CO., LTD.) 05 February 2020 (2020-02-05)<br>See entire document. | 1-15 |
| A | US 2019-0357804 A1 (IMPEDIMED LIMITED) 28 November 2019 (2019-11-28)<br>See entire document. | 1-15 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **17 June 2024** | **18 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/002477**

| Patent document cited in search report | | | Publication date (day/month/year) | Patent family member(s) | | | Publication date (day/month/year) |
|---|---|---|---|---|---|---|---|
| US | 2021-0361185 | A1 | 25 November 2021 | US | 10973431 | B2 | 13 April 2021 |
| | | | | US | 2017-0007151 | A1 | 12 January 2017 |
| | | | | WO | 2015-123603 | A1 | 20 August 2015 |
| KR | 10-2022-0009884 | A | 25 January 2022 | CN | 116133583 | A | 16 May 2023 |
| | | | | EP | 4166075 | A1 | 19 April 2023 |
| | | | | US | 2023-0144358 | A1 | 11 May 2023 |
| | | | | WO | 2022-015081 | A1 | 20 January 2022 |
| KR | 10-2022-0082909 | A | 17 June 2022 | BR | 112022007806 | A2 | 05 July 2022 |
| | | | | CA | 3155931 | A1 | 29 April 2021 |
| | | | | EP | 4048146 | A1 | 31 August 2022 |
| | | | | US | 2022-0361803 | A1 | 17 November 2022 |
| | | | | WO | 2021-081443 | A1 | 29 April 2021 |
| KR | 10-2020-0012598 | A | 05 February 2020 | CN | 110772252 | A | 11 February 2020 |
| | | | | EP | 3598939 | A1 | 29 January 2020 |
| | | | | EP | 3598939 | B1 | 07 June 2023 |
| | | | | US | 2020-0029870 | A1 | 30 January 2020 |
| US | 2019-0357804 | A1 | 28 November 2019 | AU | 2017-220382 | A1 | 06 September 2018 |
| | | | | AU | 2017-220382 | B2 | 07 April 2022 |
| | | | | CN | 109069054 | A | 21 December 2018 |
| | | | | EP | 3416549 | A1 | 26 December 2018 |
| | | | | JP | 2019-509153 | A | 04 April 2019 |
| | | | | US | 11832928 | B2 | 05 December 2023 |
| | | | | WO | 2017-139839 | A1 | 24 August 2017 |

Form PCT/ISA/210 (patent family annex) (July 2022)